# EUROPEAN PATENT SPECIFICATION

(11) **EP 1 774 338 B1**
(45) Date of publication and mention of the grant of the patent: **03.06.2009**
(21) Application number: 05760716.0
(22) Date of filing: 19.07.2005
(51) Int. Cl.: G01N 33/68, G01N 33/573

(54) **TIMP-2 AS TARGET/MARKER OF BETA CELL FAILURE**
TIMP-2 ALS ZIEL/MARKER DES VERSAGENS VON BETA-ZELLEN
TIMP-2 EN TANT QUE CIBLE/MARQUEUR DE DEFAILLANCE DE CELLULES BETA

(30) Priority: 28.07.2004 EP 04103608
(43) Date of publication of application: 18.04.2007
(73) Proprietor: F.HOFFMANN-LA ROCHE AG, 4070 Basel (CH)
(72) Inventor: CHRIST, Andreas, CH-4144 Arlesheim (CH); EVERS, Stefan, 79379 Muellheim (DE); KRAPFENBAUER, Kurt, AT-1200 Wien (AT); SEBOKOVA, Elena, CH-4103 Bottmingen (CH)
(74) Representative: Küng, Peter
(86) International application number: PCT/EP2005/007853
(87) International publication number: WO 2006/010529

(56) References cited:
- EP-A- 1 181 939
- WO-A-03/103705
- US-B1- 6 673 623
- SINGH R ET AL: "HIGH GLUCOSE DECREASES MATRIX METALLOPROTEINASE-2 ACTIVITY IN RAT MESANGIAL CELLS VIA TRANSFORMING GROWTH FACTOR-BETA1" EXPERIMENTAL NEPHROLOGY, KARGER, DE, vol. 9, no. 4, 2001, pages 249-257, XP008052773 ISSN: 1018-7782

### Sequence listing

<110> F. Hoffmann-La Roche AG
<120> TIMP-2 as a novel target/marker for beta-cell failure
<130> case 22663
<160> 2
<170> PatentIn version 3.2
<210> 1
   <211> 220
   <212> PRT
   <213> Homo sapiens
<220>
   <221> human TIMP-2
   <222> (1)..(220)
   <223> accession No. P16035
<400> 1
   Met Gly Ala Ala Ala Arg Thr Leu Arg Leu Ala Leu Gly Leu Leu Leu
   1               5                   10                  15
   Leu Ala Thr Leu Leu Arg Pro Ala Asp Ala Cys Ser Cys Ser Pro Val
               20                  25                  30
   His Pro Gln Gln Ala Phe Cys Asn Ala Asp Val Val Ile Arg Ala Lys
           35                  40                  45
   Ala Val Ser Glu Lys Glu Val Asp Ser Gly Asn Asp Ile Tyr Gly Asn
       50                  55                  60
   Pro Ile Lys Arg Ile Gln Tyr Glu Ile Lys Gln Ile Lys Met Phe Lys
   65                  70                  75                  80
   Gly Pro Glu Lys Asp Ile Glu Phe Ile Tyr Thr Ala Pro Ser Ser Ala
                   85                  90                  95
   Val Cys Gly Val Ser Leu Asp Val Gly Gly Lys Lys Glu Tyr Leu Ile
               100                 105                 110
   Ala Gly Lys Ala Glu Gly Asp Gly Lys Met His Ile Thr Leu Cys Asp
           115                 120                 125
   Phe Ile Val Pro Trp Asp Thr Leu Ser Thr Thr Gln Lys Lys Ser Leu
       130                 135                 140
   Asn His Arg Tyr Gln Met Gly Cys Glu Cys Lys Ile Thr Arg Cys Pro
   145                 150                 155                 160
   Met Ile Pro Cys Tyr Ile Ser Ser Pro Asp Glu Cys Leu Trp Met Asp
                   165                 170                 175
   Trp Val Thr Glu Lys Asn Ile Asn Gly His Gln Ala Lys Phe Phe Ala
               180                 185                 190
   Cys Ile Lys Arg Ser Asp Gly Ser Cys Ala Trp Tyr Arg Gly Ala Ala
           195                 200                 205
   Pro Pro Lys Gln Glu Phe Leu Asp Ile Glu Asp Pro
       210                 215                 220
<210> 2
   <211> 220
   <212> PRT
   <213> Rattus norvegicus
<220>
   <221> rat TIMP-2
   <222> (1)..(220)
   <223> accession No. P30121
<400> 2
   Met Gly Ala Ala Ala Arg Ser Leu Arg Leu Ala Leu Gly Leu Leu Leu
   1               5                   10                  15
   Leu Ala Thr Leu Leu Arg Pro Ala Asp Ala Cys Ser Cys Ser Pro Val
               20                  25                  30
   His Pro Gln Gln Ala Phe Cys Asn Ala Asp Val Val Ile Arg Ala Lys
           35                  40                  45
   Ala Val Ser Glu Lys Glu Val Asp Ser Gly Asn Asp Ile Tyr Gly Asn
       50                  55                  60
   Pro Ile Lys Arg Ile Gln Tyr Glu Ile Lys Gln Ile Lys Met Phe Lys
   65                  70                  75                  80
   Gly Pro Asp Lys Asp Ile Glu Phe Ile Tyr Thr Ala Pro Ser Ser Ala
                   85                  90                  95
   Val Cys Gly Val Ser Leu Asp Val Gly Gly Lys Lys Glu Tyr Leu Ile
               100                 105                 110
   Ala Gly Lys Ala Glu Gly Asp Gly Lys Met His Ile Thr Leu Cys Asp
           115                 120                 125
   Phe Ile Val Pro Trp Asp Thr Leu Ser Ile Thr Gln Lys Lys Ser Leu
       130                 135                 140
   Asn His Arg Tyr Gln Met Gly Cys Glu Cys Lys Ile Thr Arg Cys Pro
   145                 150                 155                 160
   Met Ile Pro Cys Tyr Ile Ser Ser Pro Asp Glu Cys Leu Trp Met Asp
                   165                 170                 175
   Trp Val Thr Glu Lys Ser Ile Asn Gly His Gln Ala Lys Phe Phe Ala
               180                 185                 190
   Cys Ile Lys Arg Ser Asp Gly Ser Cys Ala Trp Tyr Arg Gly Ala Ala
           195                 200                 205
   Pro Pro Lys Gln Glu Phe Leu Asp Ile Glu Asp Pro
       210                 215                 220

## Description

Type 2 diabetes is a disease of fast growing worldwide importance and can be described as a failure of the pancreatic beta-cell (beta-cell failure) to compensate, with enhanced insulin secretion of the beta-cells, for peripheral insulin resistance. This failure is explained by both a relative loss of beta-cell mass as well as secretory defects that include enhanced basal insulin secretion by the beta-cells and a selective loss of sensitivity to insulin mainly in skeletal muscle but also in other organs. The loss of beta-cell function is believed to be triggered by long-term exposure to enhanced levels of glucose and lipids (glyco- and lipotoxicity).

There is currently no clinically proven treatment that could prevent or delay beta-cell failure under lipo/glycotox conditions. It would also be useful to identify better targets for treatment and markers for detection of beta-cell failure or function that are more sensitive or more reliable than the markers commonly used, such as insulin, proinsulin or C-peptide.

Furthermore, it would be an advantage to identify markers that can be detected in plasma.

The aim of the present invention is to identify and provide a novel target to screen for compounds that prevent, attenuate, or inhibit beta-cell failure, and for a marker that allows for monitoring and/or diagnosis of beta-cell failure at an earlier stage of type II diabetes and more reliably than can presently be done.

Surprisingly, it was found that the use of protein TIMP-2 can overcome, at least in part, the problems known from the state of the art.

TIMP-2 interacts with the 72-kDa type IV collagenase in preference to the 92-kDa type IV collagenase that forms a complex exclusively with TIMP. TIMP-2 can inhibit the activity of 72-kDa type IV collagenase, and it is also an inhibitor of the membrane-type metalloproteinase 1. Collagenases have been implicated in cell migration and invasion in inflammation and tumor formation (see e.g. Umenishi et al., 1991, J. Biochem. 110: 189-95).

Surprisingly, it was found that changes in the levels of secreted TIMP-2 are found in beta-cell failure. Therefore, the present invention provides a target for the treatment and/or prevention of beta-cell failure, and a novel marker for the early diagnosis of beta-cell failure in diabetes.

In preferred embodiments, the novel target and/or marker TIMP-2 may be used for diagnostic, monitoring as well as for screening purposes.

When used in patient monitoring, the diagnostic method according to the present invention may help to assess efficacy of treatment and recurrence of beta-cell failure in the follow-up of patients. Therefore, the present invention provides the use of protein TIMP-1 for monitoring the efficacy of treatment of diabetes.

In a preferred embodiment, the diagnostic method according to the present invention is used for screening purposes. I.e., it is used to assess subjects without a prior diagnosis of diabetes by measuring the level of TIMP-2 and correlating the level of TIMP-2 to the presence or absence of beta cell failure.

The methods of the present invention are useful for monitoring progression of the disease through the different stages leading to diabetes, namely Insulin Resistance, Impaired Glucose Tolerance and Diabetes.

The present invention thus provides a method for monitoring the progression of diabetes, comprising the steps of (a) providing a liquid sample obtained from an individual, (b) contacting said sample with a specific binding agent for TIMP-2 under conditions appropriate for formation of a complex between said binding agent and TIMP-2, and (c) correlating the amount of complex formed in (b) to the amount of complex formed in beta-cell failure.

The present invention also provides a method for monitoring the efficacy of treatment of diabetes, comprising the steps of (a) providing a liquid sample obtained from a patient treated against diabetes, (b) contacting said sample with a specific binding agent for TIMP-2 under conditions appropriate for formation of a complex between said binding agent and TIMP-2, and (c) correlating the amount of complex formed in (b) to the amount of complex formed in the absence of treatment.

The present invention provides a method of screening for a compound which interacts with TIMP-2, comprising the steps of a) contacting protein TIMP-2 with a compound or a plurality of compounds under compositions which allow interaction of said compound or a plurality of compounds with TIMP-2; and b) detecting the interaction between said compound or plurality of compounds with said polypeptide.

The present invention provides a method of screening for a compound that prevents and/or inhibits and/or attenuates beta cell failure, comprising the steps of a) contacting a compound with protein TIMP-2; and b) measuring the activity of protein TIMP-2; wherein a compound whichstimulates or inhibits the activity of protein TIMP-2 is a compound that may prevent and/or inhibit and/or attenuate beta-cell failure. Preferably, said method additionally comprises the step of immobilizing protein TIMP-2 prior to step a) or between steps a) and b).

The term "activity" as used herein refers to functions of TIMP-2 such as, e.g., inhibition of MT-MMP1 activity by TIMP-2 (Will et al., Biol Chem. 1996 Jul 19;271(29):17119-23); or association of TIMP-2 with MT-MMP, and inhibition of gelatinase activity by TIMP-2 (Sato et al., FEBS Lett. 1996 Sep 9;393(1):101-4) e.g. by Reverse Zymography (e.g. described in Oliver et al., Anal. Biochem. 244, 161-166 (1997))

The present invention also includes cell-free assays. Such assays involve contacting a form of TIMP-2 (e.g., full-length polypeptide, a biologically active fragment of said polypeptide, or a fusion protein comprising all or a portion of said polypeptide) with a test compound and determining the ability of the test compound to bind to said polypeptide. Binding of the test compound to said polypeptide can be determined either directly or indirectly as described above. In one embodiment, the assay includes contacting the said polypeptide with a known compound which binds said polypeptide to form an assay mixture, contacting the assay mixture with a test compound, and determining the ability of the test compound to interact with said polypeptide, wherein determining the ability of the test compound to interact with said polypeptide comprises determining the ability of the test compound to preferentially bind to the said polypeptide as compared to the known compound.

The cell-free assays of the present invention are amenable to use of either a membrane-bound form of a polypeptide or a soluble fragment thereof. In the case of cell-free assays comprising the membrane-bound form of the polypeptide, it may be desirable to utilize a solubilizing agent such that the membrane-bound form of the polypeptide is maintained in solution. Examples of such solubilizing agents include nonionic detergents such as n-octylglucoside, n-dodecylglucoside, n-dodecylmaltoside, octanoyl-N-methylglucamide, decanoyl-N-methylglucamide, Triton X-100, Triton X-114, Thesit, Isotridecypoly(ethylene glycol ether)n, 3-[(3-cholamidopropyl)dimethylamminio]-1-propane sulfonate (CHAPS), 3-[(3-cholamidopropyl)dimethylamminio]-2-hydroxy-1-propane sulfonate (CHAPSO), or N-dodecyl-N, N-dimethyl-3-ammonio-1 -propane sulfonate.

In various embodiments of the above assay methods of the present invention, it may be desirable to immobilize a polypeptide to facilitate separation of complexed from uncomplexed forms of the polypeptide with a binding molecule, as well as to accommodate automation of the assay. Binding of a test compound to a polypeptide, or interaction of a polypeptide with a binding molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed binding protein or polypeptide, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of a polypeptide hereinbefore described can be determined using standard techniques.

Other techniques for immobilizing proteins on matrices can also be used in the screening assays of the invention. For example, either a polypeptide hereinbefore described or its binding molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated 96 well plates (Pierce Chemical). Alternatively, antibodies reactive with a polypeptide or binding molecules, but which do not interfere with binding of the polypeptide of the invention to its binding molecule, can be derivatized to the wells of the plate. Unbound binding protein or polypeptide of the invention is trapped in the wells by antibody conjugation. Methods for detecting such complexes, in addition to those described above for the GST-immobilized complexes, include immunodetection of complexes using antibodies reactive with a polypeptide hereinbefore described or binding molecule, as well as enzyme-linked assays which rely on detecting an enzymatic activity associated with a polypeptide or binding molecule.

The present invention also provides a method of screening for a compound that prevents and/or inhibits and/or delays beta-cell failure, comprising the step of detecting soluble TIMP-2 secreted from a host in the presence or absence of said compound, wherein a compound that prevents and/or inhibits and/or delays beta-cell failure is a compound with which the level of TIMP-2 secreted from a host is changed.

A host may be a model cell representing beta-cells in culture, or an animal which can be used as a model for beta-cell failure.

The present invention also provides for a use of protein TIMP-2 as a target and/or as a marker for screening for a compound that prevents and/or inhibits beta-cell failure.

The diagnostic, monitoring and patient screening methods according to the present invention are based on a liquid sample which is derived from an individual. Unlike to methods known from the art TIMP-2 is specifically measured from this liquid sample by use of a specific binding agent.

A specific binding agent is, e.g., a receptor for TIMP-2 or an antibody to TIMP-2. As the skilled artisan will appreciate the term specific is used to indicate that other biomolecules present in the sample do not significantly bind to the binding agent specific for TIMP-2. A level of less than 5% cross-reactivity is considered not significant.

A specific binding agent preferably is an antibody reactive with TIMP-2. The term antibody refers to a polyclonal antibody, a monoclonal antibody, fragments of such antibodies, as well as to genetic constructs comprising the binding domain of an antibody.

Antibodies are generated by state of the art procedures, e.g., as described in Tijssen (Tijssen, P., Practice and theory of enzyme immunoassays 11 (1990) the whole book, especially pages 43-78; Elsevier, Amsterdam). For the achievements as disclosed in the present invention polyclonal antibodies raised in rabbits have been used. However, clearly also polyclonal antibodies from different species, e.g. rats or guinea pigs, as well as monoclonal antibodies can also be used. Since monoclonal antibodies can be produced in any amount required with constant properties, they represent ideal tools in development of an assay for clinical routine. The generation and use of monoclonal antibodies to TIMP-2 in a method according to the present invention is yet another preferred embodiment.

As the skilled artisan will appreciate now, that TIMP-2 has been identified as a marker which is useful in the diagnosis of beta cell failure, alternative ways may be used to reach a result comparable to the achievements of the present invention. For example, alternative strategies to generate antibodies may be used. Such strategies comprise amongst others the use of synthetic peptides, representing an epitope of TIMP-2 for immunization. Alternatively, DNA immunization also known as DNA vaccination may be used.

For measurement the liquid sample obtained from an individual is contacted with the specific binding agent for TIMP-2 under conditions appropriate for formation of a binding agent TIMP-2-complex. Such conditions need not be specified, since the skilled artisan without any inventive effort can easily identify such appropriate incubation conditions.

As a final step according to the methods disclosed in the present invention the amount of complex is measured and correlated to the diagnosis of beta cell failure or to the respective control, as hereinbefore described. As the skilled artisan will appreciate there are numerous methods to measure the amount of the specific binding agent TIMP-2-complex all described in detail in relevant textbooks (cf., e.g., Tijssen P., supra, or Diamandis, et al., eds. (1996) Immunoassay, Academic Press, Boston).

Preferably TIMP-2 is detected in a sandwich type assay format. In such assay a first specific binding agent is used to capture TIMP-2 on the one side and a second specific binding agent, which is labeled to be directly or indirectly detectable, is used on the other side.

In another embodiment, TIMP-2 is measured in the liquid sample by determining the activity of TIMP-2 in said liquid sample. The activity of TIMP-2 in the liquid sample is then correlated to the activity of TIMP-2 in beta-cell failure, when monitoring the progression of diabetes, or to the activity of TIMP-2 in the absence of treatment, when monitoring the efficacy of treatment, or it is correlated to the diagnosis of beta-cell failure.

Preferably, the activity of TIMP-2 is determined using the complex of TIMP-2 with a binding agent which is formed as hereinbefore described.

As mentioned above, it has surprisingly been found that TIMP-2 can be measured from a liquid sample obtained from an individual sample. No tissue and no biopsy sample is required to apply the marker TIMP-2 in the diagnosis of beta cell failure.

In a preferred embodiment the method according to the present invention is practiced with serum as liquid sample material.

In a further preferred embodiment the method according to the present invention is practiced with plasma as liquid sample material.

In a further preferred embodiment the method according to the present invention is practiced with whole blood as liquid sample material.

Whereas application of routine proteomics methods to tissue samples, leads to the identification of many potential marker candidates for the tissue selected, the inventors of the present invention have surprisingly been able to detect protein TIMP-2 in a bodily fluid sample. Even more surprising they have been able to demonstrate that the presence of TIMP-2 in such liquid sample obtained from an individual can be correlated to the diagnosis of beta-cell failure.

Antibodies to TIMP-2 with great advantage can be used in established procedures, e.g., to beta-cell failure in situ, in biopsies, or in immunohistological procedures.

Preferably, an antibody to TIMP-2 is used in a qualitative (TIMP-2 present or absent) or quantitative (TIMP-2 amount is determined) immunoassay.

Measuring the level of protein TIMP-2 has proven very advantageous in the field of beta-cell failure and diabetes. Therefore, in a further preferred embodiment, the present invention relates to use of protein TIMP-2 as a marker molecule in the diagnosis of beta-cell failure from a liquid sample obtained from an individual.

The term marker molecule is used to indicate that changes in the level of the analyte TIMP-2 as measured from a bodily fluid of an individual marks the presence of beta cell failure.

It is preferred to use the novel marker TIMP-2 in the early diagnosis of type II diabetes.

It is especially preferred to use the novel marker TIMP-2 in the early diagnosis of glucose intolerance.

It is also especially preferred to use the novel marker TIMP-2 in the monitoring of disease progression in diabetes.

The use of protein TIMP-2 itself, represents a significant progress to the challenging field of beta-cell failure diagnosis. Combining measurements of TIMP-2 with other known markers for diabetes, like insulin, or with other markers of beta-cell failure yet to be discovered, leads to further improvements. Therefore in a further preferred embodiment the present invention relates to the use of TIMP-2 as a marker molecule for diabetes, preferably for beta-cell failure, in combination with another marker molecule for diabetes, preferably for beta-cell failure, in the diagnosis of diabetes, preferably of beta-cell failure from a liquid sample obtained from an individual. Preferred selected other diabetes markers with which the measurement of beta-cell failure may be combined are insulin, pre-insulin, and/or C-peptide.

Diagnostic reagents in the field of specific binding assays, like immunoassays, usually are best provided in the form of a kit, which comprises the specific binding agent and the auxiliary reagents required to perform the assay. The present invention therefore also relates to an immunological kit comprising at least one specific binding agent for TIMP-2 and auxiliary reagents for measurement of TIMP-2.

One way of assessing clinical utility of the novel marker TIMP-2 is by measuring its levels in 10 diabetic patients depending on injections of exogenous insulin and comparing the levels with those measured in 10 patients with demonstrated normal beta-cell function. For statistical analysis, standard Student's t-test evaluation is performed with values < 0.05 being taken as significant.

Accuracy of a test can be described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results)]. This has also been referred to as positivity in the presence of a disease or condition. It is calculated solely from the affected subgroup. On the x-axis is the false-positive fraction, or 1- specificity [defined as (number of false-positive results)/(number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Also claimed are the methods, uses and kit substantially as hereinbefore described, especially with reference to the examples below.

### Examples

In order to identify proteins secreted by INS-1 (Asfari M, Janjic D, Meda P, Li G, Halban PA, Wollheim CB. Establishment of 2-mercaptoethanol-dependent differentiated insulin-secreting cell lines. Endocrinology. 1992 Jan;130(1):167-78) or RINm5f insulinoma cells (Praz GA, Halban PA, Wollheim CB, Blondel B, Strauss AJ, Renold AE. Regulation of immunoreactive-insulin release from a rat cell line (RINm5F). Biochem J. 1983 Feb 15;210(2):345-52) we applied two methods: (i) Fractionation of the cells by differential sedimentation into sub-cellular compartments with subsequent identification of the proteins based on their peptide mass fingerprint using MALDI-TOF mass spectrometry, and (ii) enrichment of glycoproteins by heparin chromatography followed by one-dimensional SDS-PAGE and identification of proteins by analysis of the tryptic peptides resulting from protein digest by liquid chromatography coupled to tandem mass spectrometry resulting in identification based on protein sequence tags. The combination of these two purification strategies allowed us to increase the efficiency of protein identification in the cellular compartments as well as in the medium of cultured cells.

### Cell culture

We reproduced the features of beta-cell failure by chronic exposure of beta-cells to a combination of high glucose/fatty acids (FAs), suggesting that hyperlipidemia as well as hyperglycemia may contribute to decompensation of beta-cells. INS-1E and RINm5f cells pretreated for 24 h with a combination of 10 mM glucose and 0.5 mM palmitate were used for these experiments.

### Example 1

### Mapping and identification of signal proteins in cell compartment by 2DE-Electrophoresis and identification by MALDI-MS

Samples prepared from each cell line were subjected to 2-DE as described elsewhere (Peyrl A, Krapfenbauer K, Slavc I, et al., PROTEOMICS 3 (9): 1781-1800 SEP 2003; Fountoulakis M., Langen H., Anal. Biochem. 250 (1997) 153-156.). 2-DE was performed essentially as reported (Langen, H., Roeder, D., Juranville, J.-F., Fountoulakis, M., Electrophoresis 1997, 18, 2085-2090). Samples were desalted by using membrane filter tubes (Millipore, Art. No. UFV4BGC25) and 2.0 mg were applied on immobilised pH 3-10 non linear gradient strips (Amersham, Pharmacia Biotechnology, Uppsala, Sweden) at both the basic and acid ends of the strips. The proteins were focused at 200 V after which the voltage is gradually increasing to 5000 V with 2 V/min. Focusing was continued at 5000 V for 24 h. The second-dimensional separation was performed on a 12% polyacrylamide gel (Biosolve, Walkinswaard, Netherland). The gels (180 x 200 x 1.5 mm) were run at 50 mA /gels, in an Ettan DALT II system (Amersham, Pharmacia Biotechnology, Uppsala, Sweden) accommodating twelve gels. After protein fixation for 12 h in 50% methanol containing 5% phosphoric acid, the gels were stained with colloidal Coomassie blue (Novex, San Diego, CA) for 24 h. Molecular masses were determined by running standard protein markers (Gibco, Basel, Switzerland), covering the range of 10 to 200 kDa. PI values were used as given by supplier of the IPG strips (Amersham Pharmacia, Uppsala, Sweden). Gels were destained with H₂O and scanned in an AGFA DUOSCAN densitometer. Electronic images of the gels were recorded using Photoshop (Adobe) and PowerPoint (Microsoft).

*MALDI-MS*: MS analysis was performed as described (Langen, H., Roeder, D., Juranville, J.-F., Fountoulakis, M., Electrophoresis 1997, 18, 2085-2090) with minor modifications.
Briefly, spots were excised, destained with 30% (v/v) acetonitrile in 0.1 M ammonium bicarbonate and dried in a Speed vac evaporator. The dried gel pieces were reswollen with 5 µl of 5 mM ammonium bicarbonate, (pH 8.8) containing 50 ng trypsin (Promega, Madison, WI, USA) were added, centrifuged for 1 min and left at room temperature for about 12 h. After digestion, 5 µl of water was added, followed 10 min later by 10 µl 75% acetonitrile, containing 0.3% trifluoroacetic acid, was added, centrifuged for 1 min and the content was vortexed for 20 min. For MALDI-MS 1.5 µl from the separated liquid was mixed with 1 µl saturated alpha-cyano cinnamic acid in 50% acetonitril, 0.1% TFA in water and applied to the MALDI target. The samples were analysed in a time-of-flight mass spectrometer (Ultraflex, Bruker, Bremen, Germany) equipped with a reflector and delayed extraction. Des-Arg-1 Bradykinin (Sigma) and ACTH (18-38) (Sigma) were used as standard peptides. Calibration was internal to the samples. The peptide masses were matched with the theoretical peptide masses of all proteins from all species of the SWISS-Prot database.

Peak annotation for MALDI mass spectra: Mass spectrometric data is two times filtered using a low-pass median parametric spline filter in order to determine the instrument baseline. The smoothed residual mean standard deviation from the baseline is used as an estimate of the instrument noise level in the data. After baseline correction and rescaling of the data in level-over-noise coordinates, the data point with the largest deviation from the baseline is used to seed a non-linear (Levenberg-Marquardt) data fitting procedure to detect possible peptide peaks. Specifically, the fit procedure attempts to produce the best fitting average theoretical peptide isotope distribution parameterized by peak height, resolution, and monoisotopic mass. The convergence to a significant fit is determined in the usual way by tracking sigma values. After a successful convergence, an estimate for the errors of the determined parameters is produced using a bootstrap procedure using sixteen repeats with a random exchange of 1/3 of the data points. The resulting fit is subtracted from the data, the noise level in the vicinity of the fit is adjusted to the sum of the extrapolated noise level and the deviation from the peak fit, and the process is iterated to find the next peak as long as a candidate peak more than five times over level of noise can be found. The process is stopped when more than 50 data peaks have been found. The zero and first order of the time-of-flight to mass conversion are corrected using linear extrapolation from detected internal standard peaks, and confidence intervals for the monoisotopic mass values are estimated form the mass accuracies of the peaks and standards.

Probabilistic matching of spectra peaks to in-silico protein digests: Peak mass lists for mass spectra are directly compared to theoretical digests for whole protein sequence databases. For each theoretical digest, [1-Π(1- N P(pi))]^{cMatches} is calculated, where N is the number of peptides in the theoretical digest, *P(pi)* is the number of peptides that match the confidence interval for the monoisotopic mass of the peak divided by the count of all peptides in the sequence database, and *cMatches* is the number of matches between digest and mass spectrum. It can be shown that this value is proportional to the probability of obtaining a false positive match between digest and spectrum. Probability values are further filtered for high significance of the spectra peaks that produce the matches. After a first round of identifications, deviations of the identifications for mass spectra acquired under identical conditions are used to correct the second and third order terms of the time-of-flight to mass conversion. The resulting mass values have mostly absolute deviations less than 10ppm. These mass values are then used for a final round of matching, where all matches having a *Pₘᵢₛₘ* less than 0.01/NProteins (1% significance level with Bonferoni correction) are accepted.

### Example 2

### Enrichment of putative secreted proteins by heparin columns from the medium and identification by LC-MS

Based on the observation that most of the proteins with a signal function are glycosylated, the nature of Heparin Sepharose columns makes it a very versatile tool for the separation of many glycosylated proteins like e.g. proteins with signal function, growth factors, coagulation proteins and steroid receptors. The ligand in the Heparin Sepharose column is a naturally occurring sulfated glycosaminglycan which is extracted from native proteoglycan of porcine intestinal mucosa. Heparin consists of alternating units of uronic acid and D-glucosamine, most of which are substituted with one or two sulfate groups. Immobilized heparin has two main modes of interaction with proteins. It can operate as an affinity ligand; e.g. in its interaction with coagulation factors. Heparin has also a function as a high capacity cation exchanger due to its anionic sulphate groups. In our case the column was operated by using a syringe.

Recommended elution conditions for both cases comprised increasing the ionic strength by using a step gradient of 2M NaCl in which the Binding buffer was 10 mM sodium phosphate pH ∼7, and the Elution buffer was 10 mM sodium phosphate, 2 M NaCl, pH ∼7.

### Sample preparation

25 ml of the medium were centrifuged at 10.000 g, for 10 min at 4°C in order to remove cells and other insoluble materials. The sample solution was adjusted to the composition of the binding buffer. This was done by diluting the sample by adding 25 ml of a 20 mM sodium phosphate buffer solution (pH = 7). The sample was centrifuged immediately before applying it on the column. The offloading volume for the Heparin Column (HiTrap Heparin HP, 1 ml, Cat.Nr. 17-0406-01, Amersham) was 5 ml for 1 ml column.

### Operation procedure for enrichment of proteins by Heparin chromatography:

1. A 25 ml syringe was filled with binding buffer. In addition to this the stopper was removed and the column was connected to the syringe with the provided adapter "drop to drop" to avoid introducing air into column.
2. The twist-off end was removed and in order to equilibrate the column, the heparin sepharose was washed with 10 column volumes of binding buffer.
3. The sample was then prepared as described above and applied by using a syringe fitted to the luer adaptor by pumping onto the column.
4. Then, the column was washed with 5 volumes of binding buffer or until no material appeared in the effluent.
5. To elute the sample, the column was washed with 5 column volumes of elution buffer by using a step gradient.
6. Finally, the purified fractions were desalted by using POROS R2 columns.

Sample fractions eluted from the Heparin column were desalted by using reversed phase chromatography (POROS R2, PerSeptive Biosytems), and dried by using a speed vac. After drying, samples were dissolved in sample buffer mentioned below and the protein content was determined by the Bradford procedure (BioRad potein assay, BioRad).

### 1D Electrophoresis

### Sample Loading and Running Conditions

15 µg of sample were dissolved in 20 µl Sample buffer (Sample, 2.5 µl NuPAGE LDS Sample Buffer (4X), 1.0 µl NuPAGE Reducing Agent (10X), and deionized water to 6.5µl, for a total volume of 10 µl) and, before applying onto the gel, heated at 70°C for 10 minutes. The upper buffer chamber was filled with 200 ml 1X NuPAGE SDS running buffer (MES SDS running Buffer was prepared by adding 50 ml of 20X NuPAGE MES SDS Running Buffer to 950 ml deionised water). As a reducing agent, 200 µl/200 ml of the antioxidant solution was added in the upper buffer chamber. Finally, the lower buffer chamber was filled with 600 ml 1X NuPAGE SDS running buffer and gel electrophoresis was performed on a 10% BT linear gradient, polyacrylamide gels (NuPAGE, Invitrogen) at constant 200 V at RT for 35 min.

### Staining and Destaining Procedure

After protein fixing with 50% (v/v) methanol containing 5% (v/v) phosphoric acid for 12 h, the gels were stained with colloidal Coomassie blue (Novex, San Diego, CA, USA) for further 24 h. The gels were destained with H₂O and scanned in a standard flatbed scanner. The images were processed using Photoshop (Adope) and PowerPoint (Microsoft) software. Protein bands were quantified using the Image Master 2D Elite software (Amersham Pharmacia Biotechnology).

LC-MS: For identification of secreted proteins our proteomics studies were also performed using an LC/MS system named multidimensional protein identification technology (MudPIT), which combines multidimensional liquid chromatography with electro-spray ionization tandem mass spectrometry. In order to separate the digested proteins enriched by Heparin columns, our multidimensional liquid chromatography method integrates a strong cation-exchange (SCX) resin and reversed-phase resin in a biphasic column. Each MudPIT analysis was done in duplicate and separation was reproducible within 0.5% between two analyses. Furthermore, a dynamic range of 10000 to 1 between the most abundant and least abundant proteins/peptides in a complex peptide mixture has been demonstrated. By improving sample preparation along with separations, the method improved the overall analysis of proteomes by identifying proteins of a fraction enriched with secreted proteins. The MudPIT system included a 4 cm × 50 - µm i.d. × 5 µm C18 microSPE pre-column for sample concentration and an 85 cm × 15-µm i.d. × 3 µm C18 packed capillary column for high efficiency gradient reversed-phase nanoscale LC separation of extremely small sample quantities. The microSPE stage allowed solution to be loaded onto the nanoLC column at approximately 8 µL min⁻¹ which required <2 min to load a 10 µL solution with a sample loss of <5% (due to the syringe and valve adapters). The separation is conducted at a constant pressure of 10,000 psi. The long 3-µm particle packed 15-µm-i.d. capillary provides a separation peak capacity of approximately 10³. The column is connected by a zero dead volume stainless steel union fitting to a replaceable nanoESI emitter made from a 10-µm -i.d. × 150- µm-o.d. fused silica capillary with an approximately 2-µm- i.d orifice for highly efficient ionization of the eluting peptide. The ESI source is interfaced to either an FTICR MS or an ion trap MS/MS for peptide/protein detection and identification. An FTICR mass spectrometer was used for single-stage MS based upon high-accuracy mass measurements and the use of relative retention time (RRT) information, and a Finnigan ion trap mass spectrometer (LCQ XP, ThermoQuest Corp., San Jose, CA) was used for MS/MS.

### Example 3

### Generation of antibodies to the beta-cell failure marker TIMP-2

Polyclonal antibody to the beta-cell failure marker TIMP-2 is generated for further use of the antibody in the measurement of serum and plasma and blood levels of TIMP-2 by immunodetection assays, e.g. Western Blotting and ELISA.

### Recombinant protein expression in E. coli

In order to generate antibodies to TIMP-2, recombinant expression of the protein is performed for obtaining immunogens. The expression is done applying a combination of the RTS 100 expression system and E.coli. In a first step, the DNA sequence is analyzed and recommendations for high yield cDNA silent mutational variants and respective PCR-primer sequences are obtained using the "ProteoExpert RTS E.coli HY" system. This is a commercial web based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA and for in-vitro transcription and expression of the nucleotide sequence coding for the TIMP-2 protein is used. For Western-blot detection and later purification, the expressed protein contains a His-tag. The best expressing variant is identified. All steps from PCR to expression and detection are carried out according to the instructions of the manufacturer. The respective PCR product, containing all necessary T7 regulatory regions (promoter, ribosomal binding site and T7 terminator) is cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et al., Methods Enzymol. 185 (1990) 60-89) and the transformed bacteria are cultivated in a 11 batch for protein expression.

Purification of His-TIMP-2 fusion protein is done following standard procedures on a Ni-chelate column. Briefly, 11 of bacteria culture containing the expression vector for the His-TIMP-2 fusion protein is pelleted by centrifugation. The cell pellet is resuspended in lysis buffer, containing phosphate, pH 8.0, 7 M guanidium chloride, imidazole and thioglycerole, followed by homogenization using a Ultra-Turrax^{®}. Insoluble material is pelleted by high speed centrifugation and the supernatant is applied to a Ni-chelate chromatographic column. The column is washed with several bed volumes of lysis buffer followed by washes with buffer, containing phosphate, pH 8.0 and Urea. Finally, bound antigen is eluted using a phosphate buffer containing SDS under acidic conditions.

### Production of monoclonal antibodies against the protein TIMP-2

### a) Immunization of mice

12 week old A/J mice are initially immunized intraperitoneally with 100 µg TIMP-2. This is followed after 6 weeks by two further intraperitoneal immunizations at monthly intervals. In this process each mouse is administered 100 µg TIMP-2 adsorbed to aluminum hydroxide and 10⁹ germs of *Bordetella pertussis*. Subsequently the last two immunizations are carried out intravenously on the 3rd and 2nd day before fusion using 100 µg TIMP-2 in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1*10⁸ spleen cells of the immunized mouse are mixed with 2x10⁷ myeloma cells (P3X63-Ag8-653, ATCC CRL1580) and centrifuged (10 min at 300 g and 4°C.). The cells are then washed once with RPMI 1640 medium without fetal calf serum (FCS) and centrifuged again at 400 g in a 50 ml conical tube. The supernatant is discarded, the cell sediment is gently loosened by tapping, 1 ml PEG (molecular weight 4000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor. After ca. 10 days the primary cultures are tested for specific antibody. TIMP-2-positive primary cultures are cloned in 96-well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1x10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-695).

### Generation of polyclonal antibodies

### a) Immunization

For immunization, a fresh emulsion of the protein solution (100 µg/ml protein TIMP-2) and complete Freund's adjuvant at the ratio of 1:1 is prepared. Each rabbit is immunized with 1 ml of the emulsion at days 1, 7, 14 and 30, 60 and 90. Blood is drawn and resulting anti-TIMP-2 serum used for further experiments as described in examples 3 and 4.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid (25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).
The immunoglobulin in the supernatant is precipitated under vigorous stirring by the drop-wise addition of a 4 M ammonium sulfate solution to a final concentration of 2 M. The precipitated immunoglobulins are collected by centrifugation (8000 x g, 15 min, 4°C).
The supernatant is discarded. The pellet is dissolved in 10 mM NaH₂PO₄/NaOH, pH 7.5, 30 mM NaCl and exhaustively dialyzed. The dialysate is centrifuged (13,000 x g, 15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies have been biotinylated according to the same procedure.

### Digoxygenylation of polyclonal rabbit IgG

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex® 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 4

### Western Blot

Protein samples enriched and isolated from the medium by Heparin columns (mentioned above) were solved in sample buffer consisting of 10 mM Tris-HCl (pH 7.5), 150 mM NaCl, 0.05 % Tween 20,1 % SDS, and centrifuged at 12,000 g for 10 min at 4°C. The protein concentration of the supernatant was measured by Bradford using a standard curve constructed from a range of known bovine serum albumin standards. After mixing samples with sample buffer (60 mM Tris-HCl, 2% SDS, 0.1% bromphenol blue, 25% glycerol, and 14.4 mM 2-mercaptoethanol, pH 6.8) and incubation at 70°C for 5 min, samples were separated by 12.5% homogenous ExcelGel SDS gels (Amersham Bioscience) and electro transferred onto Nitrocellulose membranes. After incubation in blocking solution (10 mM Tris-HCl, pH 7.5, 150 mM NaCl, 0.05% Tween 20 and 5% non-fat dry milk), membranes were incubated with rabbit anti-rat antibody for 2 hrs at room temperature, respectively. After washing 3 times for 10 min with washing solution (0.3% Tween 20 in tris-buffered saline), membranes were incubated with a horseradish peroxidase conjugated anti-rabbit IgG (H+L), anti-mouse IgG₁ and anti-mouse IgG₂a (Southern Biotechnology Associates, Inc., Birmingham, AL), respectively, for 1 hr at room temperature. Membranes were washed 3 times for 10 min and antigen-antibody complexes were visualized by an enhanced chemiluminescence's reagent (Western Lightning ™, PerkinElmer Life Sciences, Inc., Boston, MA) on an X-ray film according to the manufacturer's protocol.

### Example 5.1

### ELISA for the measurement of TIMP-2 in human serum and plasma samples.

For detection of TIMP-2 in human serum or plasma, a sandwich ELISA is developed. For capture and detection of the antigen, aliquots of the anti-TIMP-2 polyclonal antibody (see Example 2) are conjugated with biotin and digoxygenin, respectively.

Streptavidin-coated 96-well microtiter plates are incubated with 100 µl biotinylated anti-TIMP-2 polyclonal antibody for 60 min at 10 µg/ml in 10 mM phosphate, pH 7.4, 1% BSA, 0.9% NaCl and 0.1% Tween 20. After incubation, plates are washed three times with 0.9% NaCl , 0.1% Tween 20. Wells are then incubated for 2 h with either a serial dilution of the recombinant protein (see Example 2) as standard antigen or with diluted plasma samples from patients. After binding of TIMP-2, plates are washed three times with 0.9% NaCl , 0.1% Tween 20. For specific detection of bound TIMP-2, wells are incubated with 100 µl of digoxygenylated anti-TIMP-2 polyclonal antibody for 60 min at 10 µg/ml in 10 mM phosphate, pH 7.4, 1% BSA, 0.9% NaCl and 0.1% Tween 20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4,1% BSA, 0.9% NaCl and 0.1% Tween 20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 5.2

### Validation of TIMP-2 in plasma

Validation of TIMP-2 as a biomarker for beta-cell failure was performed using the QUANTIKINE human TIMP-2 Immunoassay (R&D Systems, catalog No. DTM 200). The assay was carried out according to the manufacturer's instructions:

All reagents, working standards and samples were prepared as described. Excess microplate strips were removed from the plate frame. 100 µL of Assay Diluent RD 1W were added to each well. 50 µL of Standard, control, or sample* were added per well and the wells covered with the adhesive strip provided. The plate was incubated for 2 hours at room temperature on a horizontal orbital shaker (0.12" orbit) set at 500 ± 50 rpm. A plate layout was provided to record standards and samples assayed. Each well was aspirated and washed, repeating the process three times for a total of four washes. Complete removal of liquid at each step is essential to good performance. After the last wash, any remaining Wash Buffer was removed by aspirating or decanting. The plate was inverted and blotted against clean paper towels. 200 µL of TIMP-2 Conjugate were added to each well. The plate was covered with a new adhesive strip and incubated for 2 hours at room temperature on the shaker. The aspiration/wash steps were repeated. 200 µL of Substrate Solution were added to each well. Protect from light. Samples were incubated for 30 minutes at room temperature on the benchtop. 50 µL of Stop Solution was added to each well. The optical density of each well was determined within 30 minutes, using a microplate reader set to 450 nm.

### Example 6

### Statistical analysis of patient data:

Clinical utility of the novel marker TIMP-2 was assessed by measuring its levels in 10 diabetic patients depending on injections of exogeneous insulin and comparing the levels with those measured in 10 patients with demonstrated normal beta cell function. Statistical analysis was performed by standard Student's t-test evaluation with values <0.05 taken as significant.

The results were as follows:
Control: 2.3 ng/ml +/- 0.539 ng/ml
Type I diabetes: 3.2 ng/ml +/- 0.891 ng/ml, p=0.0107 (significant)
Type II diabetes: 2.8 ng/ml +/- 1.133 ng/ml, p=0.00000299 (highly significant)
IGT (impaired glucose tolerance): 2.9 ng/ml +/- 0.645 ng/ml, p=0.0464 (significant)
IFG (impaired fasting glucose): 2.5 ng/ml +/- 0.617 ng/ml, p=0.4798
IFG+IGT: 2.8 ng/ml +/- 0.475 ng/ml, p=0.0731

## Claims

1. A method of screening for a compound that prevents and/or inhibits and/or delays beta-cell failure, comprising the step of detecting soluble TIMP-2 secreted from a host in the presence or absence of said compound, wherein a compound that prevents and/or inhibits and/or delays beta-cell failure is a compound with which the level of TIMP-2 secreted from a host is changed.

2. Use of protein TIMP-2 as a target and/or marker for screening for a compound that prevents and/or inhibits beta cell failure.

3. A method for monitoring the progression of diabetes, comprising the steps of
a) providing a liquid sample obtained from an individual,
b) contacting said sample with a specific binding agent for TIMP-2 under conditions appropriate for formation of a complex between said binding agent and TIMP-2, and
c) correlating the amount of complex formed in (b) to the amount of complex formed in beta-cell failure.

4. A method for monitoring the efficacy of treatment of diabetes, comprising the steps of
a) providing a liquid sample obtained from a patient treated against diabetes,
b) contacting said sample with a specific binding agent for TIMP-2 under conditions appropriate for formation of a complex between said binding agent and TIMP-2, and
c) correlating the amount of complex formed in (b) to the amount of complex formed in the absence of treatment.

5. A method for the diagnosis of beta cell failure comprising the steps of
a) providing a liquid sample obtained from an individual,
b) contacting said sample with a specific binding agent for TIMP-2 under conditions appropriate for formation of a complex between said binding agent and TIMP-2, and
c) correlating the amount of complex formed in (b) to the diagnosis of beta cell failure.

6. A method for monitoring the progression of diabetes, comprising the steps of
a) providing a liquid sample obtained from an individual,
b) determining the activity of TIMP-2 in said sample;
c) correlating the activity of TIMP-2 in b) to the activity of TIMP-2 in beta-cell failure.

7. A method for monitoring the efficacy of treatment of diabetes, comprising the steps of
a) providing a liquid sample obtained from a patient treated against diabetes,
b) determining the activity of TIMP-2 in said sample;
c) correlating the activity of TIMP-2 in b) to the activity of TIMP-2 in the absence of treatment.

8. A method for diagnosis of beta-cell failure, comprising the steps of
a) providing a liquid sample obtained from an individual,
b) determining the activity of TIMP-2 in said sample;
c) correlating the activity of TIMP-2 in b) to the diagnosis of beta-cell failure.

9. The methods according to any one of claims 3 to 8, further **characterized in that** said sample is serum.

10. The method according to any one of claims 3 to 8, further **characterized in that** said sample is plasma.

11. The method according to any one of claims 3 to 8, further **characterized in that** said sample is whole blood.

12. Use of protein TIMP-2 as a marker molecule in the diagnosis of beta cell failure or in the early diagnosis of type II diabetes from a liquid sample obtained from an individual.

13. Use according to claim 12, wherein the early diagnosis is made with a sample derived from patients suffering from glucose intolerance.

14. Use of protein TIMP-2 for monitoring the progression of diabetes or for monitoring the efficacy of treatment of diabetes

15. Use of protein TIMP-2 as a marker molecule for beta cell failure in combination with at least one other marker molecule for beta cell failure in the diagnosis of beta cell failure from a liquid sample obtained from an individual.

## Patentansprüche

1. Verfahren zum Screenen auf eine Verbindung, die einem Versagen von Betazellen vorbeugt und/oder es verhindert und/oder verzögert, das den Schritt umfasst, lösliches, von einem Wirt in der Gegenwart oder Abwesenheit der Verbindung ausgeschiedenes TIMP-2 nachzuweisen, wobei eine Verbindung, die einem Versagen von Betazellen vorbeugt und/oder es verhindert und/oder verzögert, eine Verbindung ist, mit der der Spiegel von TIMP-2, das von einem Wirt ausgeschieden wird, verändert wird.

2. Verwendung des Proteins TIMP-2 als ein Ziel und/oder einen Marker zum Screenen auf eine Verbindung, die einem Versagen von Betazellen vorbeugt und/oder es verhindert.

3. Verfahren zum Überwachen des Fortschreitens von Diabetes, umfassend die Schritte
a) Bereitstellen einer flüssigen Probe, die von einem Individuum erhalten wurde,
b) Inkontaktbringen der Probe mit einem spezifischen Bindemittel für TIMP-2 unter Bedingungen, die für die Bildung eines Komplexes zwischen dem Bindemittel und TIMP-2 geeignet sind, und
c) Korrelieren der Menge des in (b) gebildeten Komplexes mit der Menge des bei Versagen von Betazellen gebildeten Komplexes.

4. Verfahren zum Überwachen der Wirksamkeit der Behandlung von Diabetes, umfassend die Schritte
a) Bereitstellen einer flüssigen Probe, die von einem Patienten, der gegen Diabetes behandelt wird, erhalten wurde,
b) Inkontaktbringen der Probe mit einem spezifischen Bindemittel für TIMP-2 unter Bedingungen, die für die Bildung eines Komplexes zwischen dem Bindemittel und TIMP-2 geeignet sind, und
c) Korrelieren der Menge des in (b) gebildeten Komplexes mit der Menge des in Abwesenheit der Behandlung gebildeten Komplexes.

5. Verfahren für die Diagnose des Versagens von Betazellen, umfassend die Schritte
a) Bereitstellen einer flüssigen Probe, die von einem Individuum erhalten wurde,
b) Inkontaktbringen der Probe mit einem spezifischen Bindemittel für TIMP-2 unter Bedingungen, die für die Bildung eines Komplexes zwischen dem Bindemittel und TIMP-2 geeignet sind, und
c) Korrelieren der Menge des in (b) gebildeten Komplexes mit der Diagnose des Versagens von Betazellen.

6. Verfahren zum Überwachen des Fortschreitens von Diabetes, umfassend die Schritte
a) Bereitstellen einer flüssigen Probe, die von einem Individuum erhalten wurde,
b) Nachweisen der Aktivität von TIMP-2 in der Probe;
c) Korrelieren der Aktivität von TIMP-2 in b) mit der Aktivität von TIMP-2 bei dem Versagen von Betazellen.

7. Verfahren zum Überwachen der Wirksamkeit der Behandlung von Diabetes, umfassend die Schritte
a) Bereitstellen einer flüssigen Probe, die von einem Patienten erhalten wurde, der gegen Diabetes behandelt wird,
b) Bestimmen der Aktivität von TIMP-2 in der Probe;
c) Korrelieren der Aktivität von TIMP-2 in b) mit der Aktivität von TIMP-2 in Abwesenheit der Behandlung.

8. Verfahren für die Diagnose des Versagens von Betazellen, umfassend die Schritte
a) Bereitstellen einer flüssigen Probe, die von einem Individuum erhalten wurde,
b) Bestimmen der Aktivität von TIMP-2 in der Probe;
c) Korrelieren der Aktivität von TIMP-2 in b) mit der Diagnose des Versagens von Betazellen.

9. Verfahren gemäß einem der Ansprüche 3 bis 8, ferner **dadurch gekennzeichnet, dass** die Probe Serum ist.

10. Verfahren gemäß einem der Ansprüche 3 bis 8, ferner **dadurch gekennzeichnet, dass** die Probe Plasma ist.

11. Verfahren gemäß einem der Ansprüche 3 bis 8, ferner **dadurch gekennzeichnet, dass** die Probe Vollblut ist.

12. Verwendung des Proteins TIMP-2 als ein Markermolekül in der Diagnose von Versagen von Betazellen oder in der frühen Diagnose von Typ II-Diabetes von einer flüssigen Probe, die von einem Individuum erhalten wurde.

13. Verwendung gemäß Anspruch 12, wobei die frühe Diagnose mit einer Probe durchgeführt wird, die von Patienten stammt, die an Glucose-Intoleranz leiden.

14. Verwendung des Proteins TIMP-2 zum Überwachen des Fortschreitens von Diabetes oder zum Überwachen der Wirksamkeit einer Diabetes-Behandlung.

15. Verwendung des Proteins TIMP-2 als ein Markermolekül für das Versagen von Betazellen in Kombination mit mindestens einem anderen Markermolekül für das Versagen von Betazellen in der Diagnose des Versagens von Betazellen von einer flüssigen Probe, die von einem Individuum erhalten wurde.

## Revendications

1. Procédé pour sélectionner un composé qui prévient et/ou inhibe et/ou retarde l'insuffisance des cellules bêta, comprenant l'étape de détection de TIMP-2 soluble sécrétée par un hôte en présence ou en l'absence dudit composé, où un composé qui prévient et/ou inhibe et/ou retarde l'insuffisance des cellules bêta est un composé avec lequel le niveau de TIMP-2 sécrétée par un hôte est modifié.

2. Utilisation de la protéine TIMP-2 comme cible et/ou marqueur pour sélectionner un composé qui prévient et/ou inhibe l'insuffisance des cellules bêta.

3. Procédé pour surveiller l'évolution du diabète, comprenant les étapes de :
a) fournir un échantillon liquide obtenu auprès d'un individu,
b) mettre en contact ledit échantillon avec un agent de liaison spécifique pour TIMP-2 dans des conditions appropriées pour la formation d'un complexe entre ledit agent de liaison et TIMP-2, et
c) corréler la quantité de complexe formée en (b) à la quantité de complexe formée en cas d'insuffisance des cellules bêta.

4. Procédé pour surveiller l'efficacité d'un traitement du diabète, comprenant les étapes de :
a) fournir un échantillon liquide obtenu auprès d'un patient traité contre le diabète,
b) mettre en contact ledit échantillon avec un agent de liaison spécifique pour TIMP-2 dans des conditions appropriées pour la formation d'un complexe entre ledit agent de liaison et TIMP-2, et
c) corréler la quantité de complexe formée en (b) à la quantité de complexe formée en l'absence de traitement.

5. Procédé pour le diagnostic d'une insuffisance des cellules bêta comprenant les étapes de :
a) fournir un échantillon liquide obtenu auprès d'un individu,
b) mettre en contact ledit échantillon avec un agent de liaison spécifique pour TIMP-2 dans des conditions appropriées pour la formation d'un complexe entre ledit agent de liaison et TIMP-2, et
c) corréler la quantité de complexe formée en (b) au diagnostic d'insuffisance des cellules bêta.

6. Procédé pour surveiller l'évolution du diabète, comprenant les étapes de :
a) fournir un échantillon liquide obtenu auprès d'un individu,
b) déterminer l'activité de TIMP-2 dans ledit échantillon ;
c) corréler l'activité de TIMP-2 en b) à l'activité de TIMP-2 en cas d'insuffisance des cellules bêta.

7. Procédé pour surveiller l'efficacité d'un traitement du diabète, comprenant les étapes de :
a) fournir un échantillon liquide obtenu auprès d'un patient traité contre le diabète,
b) déterminer l'activité de TIMP-2 dans ledit échantillon ;
c) corréler l'activité de TIMP-2 en b) à l'activité de TIMP-2 en l'absence de traitement.

8. Procédé pour le diagnostic d'une insuffisance des cellules bêta, comprenant les étapes de :
a) fournir un échantillon liquide obtenu auprès d'un individu,
b) déterminer l'activité de TIMP-2 dans ledit échantillon ;
c) corréler l'activité de TIMP-2 en b) au diagnostic d'insuffisance des cellules bêta.

9. Procédé selon l'une quelconque des revendications 3 à 8 **caractérisé en outre en ce que** ledit échantillon est du sérum.

10. Procédé selon l'une quelconque des revendications 3 à 8 **caractérisé en outre en ce que** ledit échantillon est du plasma.

11. Procédé selon l'une quelconque des revendications 3 à 8 **caractérisé en outre en ce que** ledit échantillon est du sang total.

12. Utilisation de la protéine TIMP-2 comme molécule marqueur dans le diagnostic d'une insuffisance des cellules bêta ou dans le diagnostic précoce du diabète de type II à partir d'un échantillon liquide obtenu auprès d'un individu.

13. Utilisation selon la revendication 12 où le diagnostic précoce est établi avec un échantillon issu de patients souffrant d'une intolérance au glucose.

14. Utilisation de la protéine TIMP-2 pour surveiller l'évolution du diabète ou pour surveiller l'efficacité d'un traitement du diabète.

15. Utilisation de la protéine TIMP-2 comme molécule marqueur pour une insuffisance des cellules bêta en combinaison avec au moins une autre molécule marqueur pour une insuffisance des cellules bêta dans le diagnostic d'une insuffisance des cellules bêta à partir d'un échantillon liquide obtenu auprès d'un individu.
